(19) Europäisches Patentamt
European Patent Office
Office européen des brevets

(11) Veröffentlichungsnummer: **0 022 533**
B2

(12) # NEUE EUROPÄISCHE PATENTSCHRIFT

(45) Veröffentlichungstag der neuen Patentschrift: **02.04.86**

(51) Int. Cl.⁴: **C 07 C 39/08,** C 07 C 37/60, C 07 C 37/82

(21) Anmeldenummer: **80103851.4**

(22) Anmeldetag: **07.07.80**

(54) **Verfahren zur Herstellung mehrwertiger Phenole.**

(30) Priorität: **17.07.79 DE 2928743**

(43) Veröffentlichungstag der Anmeldung:
**21.01.81 Patentblatt 81/3**

(45) Bekanntmachung des Hinweises auf die Patenterteilung:
**28.07.82 Patentblatt 82/30**

(45) Bekanntmachung des Hinweises auf die Entscheidung über den Einspruch:
**02.04.86 Patentblatt 86/14**

(84) Benannte Vertragsstaaten:
**BE CH DE FR GB IT LI NL**

(56) Entgegenhaltungen:
**DD - A - 134 427**
**DE - A - 1 668 952**
**DE - A - 2 064 497**
**DE - A - 2 658 866**
**FR - A - 2 071 464**
**US - A - 3 179 703**

**J. Varagnet - Ind. Eng. Chem. Prod. Res-Div. vol. 15, no. 3, (1976), S. 212-215**
**A.T. MENYALLO et al., Khim. Prom. 46 (1970) 92-4 (C.A. 73 (3598 u.)**
**A.T. MENYALLO et al. Ibid 48 (1972) 816-18 (C.A. 78(57915s)**

(73) Patentinhaber: **BAYER AG, Konzernverwaltung RP Patentabteilung, D-5090 Leverkusen 1 Bayerwerk (DE)**

(72) Erfinder: **Jupe, Christoph, Dr., Franz-Peter-Kürten-Weg 2, D-5000 Köln 80 (DE)**
Erfinder: **Waldmann, Helmut, Dr., Carl-Rumpff-Strasse 59, D-5090 Leverkusen 1 (DE)**
Erfinder: **Seifert, Hermann, Dr., Ruwergasse 4, D-5000 Köln 80 (DE)**

(56) Entgegenhaltungen: (Fortsetzung)
**L.A. SHILOV et al. Khim. Prom. st. 1975, 183-4 (C.A.82(155634a)**
**Certificat d'auteur Soviétique no. 265.104**

**Die Akte enthält technische Angaben, die nach dem Eingang der Anmeldung eingereicht wurden und die nicht in dieser Patentschrift enthalten sind.**

**Beschreibung**

Die vorliegende Erfindung betrifft ein verbessertes Verfahren zur Herstellung mehrwertiger Phenole durch Hydroxylierung von Phenolen.

Bei der Hydroxylierung von Phenolen werden eine oder mehrere Hydroxylgruppen in Phenole eingeführt, wobei aromatische Verbindungen entstehen, die zwei oder mehr direkt an einen oder mehrere aromatische Kerne gebundene Hydroxylgruppen besitzen (= mehrwertige Phenole). Die wichtigsten mehrwertigen Phenole sind solche, die zwei Hydroxylgruppen an einem aromatischen Kern besitzen und sich von Phenol, Naphthalin, Anthracen und Phenanthren ableiten. Diese zweiwertigen Phenole stellen technisch wichtige Verbindungen dar, die in grossen Mengen hergestellt werden und beispielsweise auf dem Gebiet der Photographie, der Farb- und Kunststoffe, der Geruchs- und Geschmacksstoffe, insbesondere als Zwischenprodukte auf diesen Gebieten verwendet werden (s. z.B. Kirk-Othmer, Encyclopedia of Chemical Technology, Second Edition, Vol. 11, Seiten 462 bis 492, insbesondere Seiten 469 und 488 [1966]).

Es sind eine Reihe von Verfahren zur Herstellung von mehrwertigen Phenolen bekannt, bei denen mehrwertige Phenole durch Hydroxylierung von Phenolen erhalten werden. Als Hydroxylierungsmittel werden dabei häufig peroxidische Verbindungen verwendet, beispielsweise Wasserstoffperoxid, Peroxosalze oder Persäuren, insbesondere Percarbonsäuren (s. z.B. DE-AS 2 064 497, DE-AS 1 593 968, DE-AS 1 543 830, DE-AS 2 364 181, DE-AS 2 407 398, DE-OS 2 658 866 und DE-OS 2 658 866 und DE-OS 2 658 943).

Bei diesen Verfahren werden im allgemeinen die zu hydroxylierenden Phenole in grossem Überschuss, bezogen auf das peroxidische Hydroxylierungsmittel, eingesetzt, d.h. die zu hydroxylierenden Phenole werden nur zum Teil hydroxyliert. So wird z.B. in der DE-OS 1 593 968 beschrieben, dass ein Umwandlungsgrad des eingesetzten Phenols von 30% nicht überschritten werden soll, da sonst die Ausbeute an mehrwertigen Phenolen, in diesem Fall Diphenolen, sehr rasch abnimmt. In der DE-OS 2 364 181 wird beschrieben, dass bei der Anwendung von Persäuren als Hydroxylierungsmittel für Phenole zweckmässigerweise nicht mehr als 0,5 Mol Persäure pro Mol Phenol eingesetzt wird, da grössere Mengen an diesem Oxidationsmittel eine sekundäre Oxidation bewirken, welche die Ausbeute des gewünschten Produktes in extremen Ausmass vermindert.

Bei der technischen Durchführung solcher Verfahren werden deshalb nach der Hydroxylierungsreaktion die nicht umgesetzten Anteile der eingesetzten Phenole zurückgewonnen und wieder in die Hydroxylierungsreaktion eingesetzt. Gegebenenfalls werden den rückgeführten Phenolen soviel frische Phenole zugefügt, wie in der Hydroxylierungsreaktion umgesetzt worden sind. Ein derartiges technisches Verfahren zur Herstellung von Brenzcatechin und Hydrochinon aus Phenol und Wasserstoffperoxid ist beispielsweise beschrieben in Ind. Eng. Chem. Prod. Res. Div., Vol. 15, Nr. 3 (1976), S. 212-215.

Es wurde nun festgestellt, dass, sowohl bei der ausschliesslichen Verwendung von frischen Phenolen, insbesondere aber bei der Verwendung von rückgeführten Phenolen, die Selektivität der Bildung von mehrwertigen Phenolen nicht befriedigend ist.

In der FR-PS 2 071 464 wird ein Verfahren beschrieben, bei dem während der Hydroxylierung von aromatischen Verbindungen mit Wasserstoffperoxid eine katalytische Menge einer starken Säure zugegen ist, beispielsweise ein sulfoniertes Harz. Dieser Literaturstelle kann jedoch kein Hinweis darauf entnommen werden, wie es sich auswirkt, wenn das zu hydroxylierende Gemisch ganz oder teilweise vor der Hydroxylierung mit Ionenaustauschern behandelt wird.

Es wurde nun ein Verfahren zur Herstellung mehrwertiger Phenole durch Hydroxylierung von Phenolen mit Percarbonsäuren gefunden, das dadurch gekennzeichnet ist, dass man die zu hydroxylierenden Phenole in geschmolzener oder gelöster Form vor der Hydroxylierung ganz oder teilweise mit Ionenaustauschern behandelt, die saure und gegebenenfalls zusätzlich basische Gruppen enthalten.

Für das erfindungsgemässe Verfahren kommen beispielsweise flüssige oder feste Ionenaustauscher in Frage, die saure und gegebenenfalls zusätzlich basische Gruppen enthalten. Insbesondere kommen derartige Ionenaustauscher in Frage, die aus organischen oder anorganischen Polymeren aufgebaut sind. Beispielsweise können die Ionenaustauscher aus organischen Harzen auf der Basis von Vinylbenzol, Divinylbenzol, Vinylalkohol, (Meth)Acrylsäure, Ethylen oder Perfluorethylen oder Mischpolymerisaten davon aufgebaut sein. Die Ionenaustauscher können auch aus organischen Polymeren aufgebaut sein auf der Basis eines oder mehrerer der vorgenannten Stoffe in Kombination mit anderen polymerisierbaren Substanzen, beispielsweise in Kombination mit Styrol. Als anorganische Ionenaustauscher sind beispielsweise solche auf Silikatbasis geeignet.

Erfindungsgemäss einsetzbare Ionenaustauscher enthalten als funktionelle Gruppen saure Gruppen, beispielsweise $HSO_3$- und/oder HOOC-Gruppen. Zusätzlich können sie als funktionelle Gruppen basische Gruppen enthalten, beispielsweise $NH_2$-, NH-Alkyl-, N-Dialkyl-, N-Alkyl-hydroxyalkyl- und/oder N-Diaryl-Gruppen. Die funktionellen Gruppen können in freier Form oder ganz oder teilweise in Salzform vorliegen.

Es ist auch möglich, verschiedene der vorstehend beschriebenen Ionenaustauscher in beliebiger Reihenfolge nacheinander oder in beliebiger Mischung zu verwenden.

Ionenaustauscher, welche die vorstehenden Merkmale aufweisen, sind im Handel erhältlich.

Bevorzugt werden in das erfindungsgemässe Verfahren Ionenaustauscher eingesetzt, die aus

einem Grundpolymerisat (Matrix) aus Styrol-Divinylbenzol, Styrol-Acrylsäure, Divinylbenzol-Acrylsäure, Styrol-Methacrylsäure oder Divinylbenzol-Methacrylsäure aufgebaut sind und die als funktionelle Gruppen $HSO_3$- und/oder HOOC-Gruppen enthalten. Gegebenenfalls können als zusätzliche funktionelle Gruppen $N(CH_3)_2$-, $N(CH_3)(CH_2$-$CH_2$-OH) und/oder $\overset{\oplus}{N}(CH_3)_2(CH_2$-$CH_2$-OH)-Gruppen enthalten sein. Auch bei den bevorzugt einzusetzenden Ionenaustauschern können die funktionellen Gruppen in freier Form oder ganz oder teilweise in Salzform vorliegen. Ein Austauscherharz, das als funktionelle Gruppen Aminocarbonsäurereste enthält, eignet sich ebenfalls gut für das erfindungsgemässe Verfahren.

Besonders bevorzugt werden feste Ionenaustauscher auf der Basis Acrylsäure-Divinylbenzol eingesetzt, wobei die HOOC-Gruppen in freier Form oder ganz oder teilweise in Form ihrer Metallsalze, insbesondere ihrer Erdalkali- und/oder Alkalisalze, vorliegen.

Als zu hydroxylierende Phenole, die erfindungsgemäss vor der Hydroxylierungsreaktion ganz oder teilweise mit Ionenaustauschern behandelt werden, kommen die verschiedensten aromatischen Verbindungen in Frage, die eine oder mehrere an einen oder mehrere aromatische Kerne gebundene Hydroxylgruppen enthalten und in die eine oder mehrere Hydroxylgruppen mit peroxidischen Hydroxylierungsmitteln eingeführt werden können. Im folgenden werden diese Verbindungen Phenole genannt.

Die Phenole können ausser einer oder mehreren Hydroxylgruppen keine weiteren Substituenten aufweisen, sie können jedoch auch verschiedene Substituenten tragen, soweit diese nicht die Hydroxylierungsreaktion verhindern. Als Phenole kommen beispielsweise aromatische Hydroxyverbindungen in Frage, die sich von Benzol, Naphthalin, Phenanthren oder Anthracen ableiten und die noch mindestens ein freies Wasserstoffatom an einem aromatischen Kern aufweisen. Bei Phenolen, die sich von Benzol ableiten, befindet sich ein freies Wasserstoffatom bevorzugt in 2- oder 4-Stellung zu einer bereits vorhandenen Hydroxylgruppe. Die Phenole können ausser einer oder mehreren Hydroxylgruppen an dem oder den aromatischen Kernen als Substituenten beispielsweise einen oder mehrere, gleiche oder verschiedene aliphatische, cycloaliphatische, Phenyl- oder Naphthylreste enthalten. Als aliphatische Reste kommen beispielsweise geradkettige und verzweigte Reste mit beispielsweise 1 bis 10 C-Atomen in Frage, wie Methyl, Ethyl, Isopropyl, n-Butyl, i-Butyl, tert.-Butyl, n-Pentyl, 2-Methylbutyl, 3-Methylbutyl, 2-Ethylpropyl, n-Hexyl, 2-Methylpentyl, 3-Methylpentyl, 4-Methylpentyl, n-Heptyl, n-Octyl, 2-Ethylhexyl, n-Nonyl, 2-Ethylheptyl und n-Decyl. Als cycloaliphatische Reste kommen beispielsweise solche mit 3 bis 12 C-Atomen in Frage, wie Cyclopropyl, Cyclobutyl, Cyclopentyl, Cyclohexyl, Cycloheptyl, Cyclooctyl, Cyclononyl und Cyclodecyl.

In den aliphatischen, cycloaliphatischen, Phenyl- und Naphthylresten können jeweils ein oder mehrere Wasserstoffatome durch Gruppen ersetzt sein, die unter den Bedingungen der Hydroxylierungsreaktion stabil sind. Beispiele für derartige Gruppen sind eines oder mehrere Fluor-, Chlor- und/oder Bromatome; eine oder mehrere $C_1$- bis $C_5$-Alkoxy-, $C_1$- bis $C_5$-Dialkylamino-, Carboxy-, Nitro-, Cyan-, Sulfonsäure- und/oder Carboalkoxygruppen, deren Alkoxyreste 1 bis 10 C-Atome aufweisen.

Auch der oder die aromatischen Kerne der Phenole können durch eine oder mehrere dieser Atome bzw. Gruppen substituiert sein.

Bevorzugt werden in das erfindungsgemässe Verfahren Phenole eingeführt, die sich vom Benzol ableiten.

Beispielsweise können in das erfindungsgemässe Verfahren eingesetzt werden Phenole der Formel

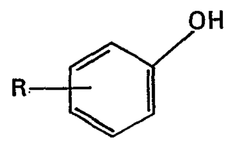

(I)

worin

R für Wasserstoff, $C_1$- bis $C_{10}$-Alkyl, $C_3$- bis $C_{12}$-Cycloalkyl, Phenyl, Naphthyl, Fluor, Chlor, Brom, eine Nitro-, Cyan-, Sulfonsäure-, Carbohydroxy-, Carbo-$C_1$- bis $C_{10}$-Alkoxy-, $C_1$- bis $C_3$-Alkoxy- oder $C_1$- bis $C_4$-Dialkylamino-Gruppe steht, wobei die Alkyl- und Cycloalkyl-Reste durch Fluor-, Chlor-, Bromatome, $C_1$- bis $C_5$-Alkoxy-, $C_1$- bis $C_4$-Dialkylamino, Carboxy-, Nitro-, Cyan-, Sulfosäure- oder $C_1$- bis $C_{10}$-Carbalkoxy-Gruppen und die Phenyl- und Naphthyl-Reste durch Fluor, Chlor, Brom, $C_1$- bis $C_{10}$-Alkyl, $C_3$- bis $C_{12}$-Cycloalkyl, sowie Nitro-, Carbohydroxy-, Carbo-$C_1$- bis $C_{10}$-Alkoxy-, $C_1$- bis $C_5$-Alkoxy-, Cyan-, Sulfonsäure- oder $C_1$- bis $C_4$-Dialkylamino-Gruppen substituiert sein können.

Eine bevorzugte Gruppe von Phenolen innerhalb der Formel (I) entspricht den Phenolen der Formel

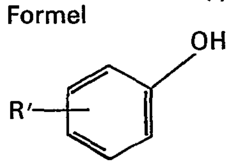

(II)

worin

R′ für Wasserstoff, $C_1$- bis $C_3$-Alkyl, $C_5$- bis $C_7$-Cycloalkyl, Phenyl, Fluor, Chlor, eine Nitro-, Sulfonsäure-, Carbohydroxy-, Carbo-$C_1$- bis $C_3$-alkoxy-, $C_1$- bis $C_2$-Alkoxy- oder $C_1$- bis $C_2$-Dialkylamino-Gruppe steht.

Besonders bevorzugt setzt man in das erfindungsgemässe Verfahren einwertige Phenole der Formel

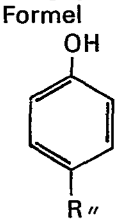

(III)

ein,

worin

R″ für Wasserstoff, $C_1$- bis $C_5$-Alkyl, $C_5$- bis $C_7$-Cycloalkyl, Phenyl, Fluor, Chlor, eine Nitro-, Sulfonsäure-, Carbohydroxy-, Carbo-$C_1$- bis $C_3$-alkoxy-, $C_1$- bis $C_3$-Alkoxy- oder $C_1$- bis $C_2$-Dialkylamino-Gruppe bedeutet.

Im einzelnen seien als Phenole, die zum Einsatz in das erfindungsgemässe Verfahren geeignet sind, beispielsweise genannt: Phenol, o-Kresol, m-Kresol, p-Kresol, p-Cyclohexylphenol, o-Cyclohexylphenol, p-Phenylphenol, o-Phenylphenol, m-Phenylphenol, p-Ethylphenol, o-Ethylphenol, m-Ethylphenol, o-Isopropylphenol, p-Isopropylphenol, o- und tert.-Butylphenol, p-Nitrophenol, o-Nitrophenol, m-Nitrophenol, 2-Brom-4-methylphenol, p-Chlorphenol, o-Chlorphenol, m-Chlorphenol, p-Carbomethoxyphenol, Salicylsäureethylester, p-Cyclopentylphenol, o-Dimethylaminophenol, p-Cyano-phenol, p-Methoxyphenol, o-Isopropoxyphenol, p-Ethoxy-phenol, 3,5-Diethyl-phenol, Thymol, Phenol, Carvacrol, 1,2,3-Xylenol, 1,2,4-Xylenol, 1,3,2-Xylenol, 1,3,4-Xylenol, 1,3,5-Xylenol, 1,4,2-Xylenol, α-Naphthol, β-Naphthol, 1-Hydroxy-4-methylnaphthalin, 1-Hydroxy-2-methylnaphthalin, 2-Hydroxy-1-methylnaphthalin, 2-Hydroxy-6-methylnaphthalin, 1-Hydroxy-4-isopropylnaphthalin, 1-Hydroxy-4-t-butylnaphthalin, 1-Hydroxy-6-phenylnaphthalin, 1-Hydroxy-6-methoxy-naphthalin, 1-Hydroxy-anthracen, 2-Hydroxy-anthracen.

Ganz besonders bevorzugt setzt man in das erfindungsgemässe Verfahren Phenole ($C_6H_5OH$) ein.

Das erfindungsgemässe Verfahren kann auf zu hydroxylierende Phenole angewendet werden, die bei der Hydroxylierungsreaktion nicht umgesetzt und abgetrennt wurden und in die Hydroxylierungsreaktion zurückgeführt werden (im folgenden als Rückphenole bezeichnet) und auf frische Phenole, die in die Hydroxylierungsreaktion eingesetzt werden (im folgenden als Frischphenole bezeichnet). Im allgemeinen ist es vorteilhaft, die gesamte Menge an Rück- und Frischphenolen zu vereinigen, sie dem erfindungsgemässen Verfahren gemeinsam zu unterwerfen und sie dann gemeinsam der Hydroxylierungsreaktion zuzuführen. Man kann auch die gesamte Menge Rückphenole und die gesamte Menge Frischphenole separat dem erfindungsgemässen Verfahren unterwerfen und dann vereinigt oder getrennt der Hydroxylierungsreaktion zuführen. In gewissen Fällen werden auch gute Ergebnisse erzielt, wenn man nur die Rückphenole oder nur die Frischphenole dem erfindungsgemässen Verfahren unterwirft. Es ist in gewissen Fällen möglich, auch nur einen Teil der Rückphenole und/oder der Frischphenole oder einen Teil der Mischung aus Rückphenolen und Frischphenolen dem erfindungsgemässen Verfahren zu unterwerfen.

Wenn man nicht die gesamten Frisch- und Rückphenole dem erfindungsgemässen Verfahren unterwerfen will, ist es im allgemeinen vorteilhaft, durch Vorversuche, gegebenenfalls im verkleinerten Massstab, festzustellen, welchen Anteil der Frisch- und/oder Rückphenole man von der Behandlung gemäss dem erfindungsgemässen Verfahren ausschliessen kann, ohne dass sich die Selektivität der Bildung von hydroxylierten Phenolen zu sehr erniedrigt. In vielen Fällen ist es nicht möglich, dies durch analytische Untersuchungen der Phenole festzustellen.

Bei der Durchführung des erfindungsgemässen Verfahrens geht man so vor, dass man die zu behandelnden Phenole in geschmolzener oder gelöster Form mit dem oder den Ionenaustauschern behandelt. Die Phenole werden vorteilhafterweise in gelöster Form mit den Ionenaustauschern in Kontakt gebracht, wenn sie Schmelzpunkte über 120°C aufweisen oder, wenn die gewünschte Behandlungstemperatur unterhalb ihres Schmelzpunkts liegt. Als Lösungsmittel für die Phenole kommen prinzipiell alle Lösungsmittel in Frage, die hinreichend inert sind und ein ausreichendes Lösevermögen für die Phenole aufweisen. Vorzugsweise verwendet man als Lösungsmittel solche, die auch in der Hydroxylierungsreaktion eingesetzt werden. Beispielsweise kann man organische Lösungsmittel, wie Benzol, chlorierte Kohlenwasserstoffe oder Carbonsäureester verwenden, wenn die Hydroxylierung mit Percarbonsäuren vorgenommen werden soll, die in Benzol, chlorierten Kohlenwasserstoffen oder Carbonsäureestern gelöst sind.

Die erfindungsgemässe Behandlung mit Ionenaustauschern kann bei unterschiedlichen Temperaturen vorgenommen werden. Im allgemeinen kann man bei Temperaturen im Bereich von 0 bis 200°C arbeiten. Vorzugsweise arbeitet man bei Temperaturen im Bereich 20 bis 120°C, besonders bevorzugt im Bereich von 40 bis 100°C. Zu beachten ist in jedem Fall, dass die Behandlungstemperatur nicht oberhalb derjenigen Temperatur liegt, bei welcher der oder die verwendeten Ionenaustauscher beginnen unbeständig zu werden.

Die verfahrenstechnische Durchführung des erfindungsgemässen Verfahrens kann in an sich bekannter Weise erfolgen. Beispielsweise sind alle Arbeitsweisen geeignet, die beim Einsatz der beschriebenen Ionenaustauscher üblicherweise angewendet werden. So können feste, als Granulat oder in beliebiger Körnung vorliegende Ionenaustauscher in die geschmolzenen oder gelösten Phenole eingerührt und nach der Behandlung mechanisch abgetrennt werden. Vorzugsweise wendet man die als Fest-, Fliess- oder Schwebebettverfahren bekannten Arbeitsweisen an, besonders bevorzugt das Festbettverfahren, bei dem die geschmolzenen oder gelösten Phenole in einer Säule über einen festen Ionenaustauscher strömen.

Die Zeit für die Behandlung gemäss dem erfindungsgemässen Verfahren kann in weiten Grenzen variiert werden. Im allgemeinen sind Behandlungszeiten im Bereich von 1 bis 300 Minuten ausreichend. Vorzugsweise beträgt die Behandlungszeit 5 bis 180 Minuten, besonders bevorzugt 10 bis 120 Minuten.

Da die Phenole im allgemeinen empfindlich gegen Sauerstoff sind, ist es im allgemeinen vorteilhaft, vor, während und nach der erfindungsgemässen Behandlung die Gegenwart von Luft bzw. Sauerstoff auszuschliessen, beispielsweise durch Überlagerung mit einem inerten Gas wie Stickstoff.

Das Mengenverhältnis zwischen Ionenaustauscher und Phenolen kann ebenfalls in weiten Grenzen variiert werden. Man kann beispielsweise bei diskontinuierlicher Arbeitsweise 1 Gew.-Teil Ionenaustauscher zur Behandlung von 1 bis 1000 Gew.-Teilen Phenolen verwenden. Durch mehrfache Verwendung lässt sich die Ausnützung des Ionenaustauschers noch erheblich steigern.

Wenn man eine kontinuierliche Arbeitsweise anwendet, z.B. indem man geschmolzene oder gelöste Phenole über einen stationär angeordneten Ionenaustauscher strömen lässt, kann man beispielsweise die Menge an Ionenaustauscher bzw. die Strömungsgeschwindigkeit so wählen, dass pro Stunde ein Gew.-Teil Ionenaustauscher mit 0,1 bis 1000 Gew.-Teilen Phenolen in Kontakt gebracht wird. Vorzugsweise bringt man dabei pro Stunde 1 Gew.-Teil Ionenaustauscher mit 0,5 bis 300, besonders bevorzugt mit 1 bis 20 Gew.-Teilen Phenolen, in Kontakt.

Ionenaustauscher können im allgemeinen bei diskontinuierlicher Arbeitsweise mehrfach bzw. bei kontinuierlicher Arbeitsweise über längere Zeit verwendet werden. Beispielsweise kann man insgesamt bis zu $10^8$ Gew.-Teile Phenole mit 1 Gew.-Teil Ionenaustauscher behandeln.

Im erfindungsgemässen Verfahren gebrauchte Ionenaustauscher können in an sich bekannter Weise durch die übliche Behandlung mit Wasser, Säure und/oder Lauge regeneriert und dann erneut im erfindungsgemässen Verfahren eingesetzt werden.

Das erfindungsgemässe Verfahren wird bei der Hydroxylierung von Phenolen mit Percarbonsäuren angewendet.

Vorzugsweise wird das erfindungsgemässe Verfahren bei folgender Hydroxylierung angewendet:

– Bei der Herstellung von mehrwertigen Phenolen durch Hydroxylierung von Phenolen mit einer weitgehend wasser- und wasserstoffperoxidfreien Lösung einer Percarbonsäure in einem organischen Lösungsmittel (s. DE-OS 2 658 943). Die Percarbonsäure enthält dabei vorzugsweise 1 bis 4 C-Atome und das organische Lösungsmittel ist dabei beispielsweise Benzol, 1,2-Dichlorpropan oder Essigsäureethylester.

Bei einer möglichen technischen Ausführungsform des erfindungsgemässen Verfahrens kann wie folgt gearbeitet werden:

Das aus der Aufarbeitung von bereits hydroxyliertem Phenol stammende Phenol wird bei 50 bis 90°C kontinuierlich über einen sauren Ionenaustauscher auf der Basis eines sulfonierten Styrol-/Divinylbenzol-Copolymerisates oder eines Acrylsäureharzes geleitet, so dass sich eine mittlere Verweilzeit von 5 bis 60 Minuten ergibt. Diesem Phenolstrom wird, vor oder nach der Behandlung mit Ionenaustauscher, die durch Reaktion verbrauchte Phenolmenge zugesetzt. Das so erhaltene Einsatzphenol-Gemisch wird kontinuierlich einer Anordnung von Reaktionsgefässen zugeführt, in die neben dem Phenolgemisch gleichzeitig kontinuierlich die Lösung einer Percarbonsäure mit 1 bis 4 C-Atomen in einem inerten organischen Lösungsmittel, wie 1,2-Dichlorpropan oder Benzol oder Essigsäureethylester, zugegeben wird. Das Reaktionsgemisch, das überwiegend aus Phenol, dem organischen Lösungsmittel, der entsprechenden Carbonsäure mit 1 bis 4 C-Atomen und den Reaktionsprodukten Brenzkatechin und Hydrochinon besteht, wird nach Beendigung der Reaktion der Percarbonsäure einer destillativen Aufarbeitung zugeführt, bei der u.a. das nicht umgesetzte Phenol zurückgewonnen wird.

Das erfindungsgemässe Verfahren hat den Vorteil, dass es in einfacher Weise ohne besonderen apparativen Aufwand durchgeführt werden kann, kostspielige Trenn- und Reinigungsoperationen vermeidet und als Hilfsstoffe nur Ionenaustauscher benötigt, die lange Standzeiten haben und regeneriert werden können. Bei Anwendung des erfindungsgemässen Verfahrens erhält man hydroxylierte Phenole in höherer Selektivität als bei bekannten Verfahren, d.h. bei vergleichbaren Umsätzen von Phenolen werden hydroxilierte Phenole in höherer Ausbeute erhalten.

Es ist als ausgesprochen überraschend anzusehen, dass es mit der erfindungsgemässen Behandlung mit Ionenaustauschern gelingt, Phenole zu erhalten, die sich bei der Hydroxylierung mit Percarbonsäuren mit höherer Selektivität in hydroxylierte Phenole umwandeln lassen als nicht erfindungsgemäss behandelte Phenole.

Es ist zwar bekannt, dass man aus rohem Phenol, das Verunreinigungen enthält, die bei der Herstellung von Folgeprodukten des Phenols stören können, den Gehalt an gewissen Verunreinigungen herabsetzen kann, indem man das rohe Phenol mit bestimmten Ionenaustauschern bei bestimmten Bedingungen behandelt und das so behandelte Phenol anschliessend einer Destillation unterwirft (s. DE-OS 1 668 952, DD-PS 134 427, SU-Erfinderschein 265 104, Khim. Prom. 46, 92–94 [1970] [= C.A. 73, 3598u], ibid. 48, 816–818 [1972] [= C.A. 78, 57915s], Khim. Prom. st. 1975, 183–184 [= C.A. 82, 155634b]). Bei dieser bekannten Arbeitsweise werden durch die Behandlung mit Ionenaustauschern jedoch störende Verunreinigungen nicht entfernt, sondern Verunreinigungen, die destillativ nicht entfernbar sind (z.B. Mesityloxid) werden unter dem katalytischen Einfluss der Ionenaustauscher in Produkte umgewandelt (z.B. in höhere Kondensationsprodukte), die von Phenol destillativ abgetrennt werden können. Um die störenden Verunreinigungen bzw. ihre Folgeprodukte abzutrennen, ist immer eine Destillation nötig. Zusätzlich ist es bei diesem bekannten Verfahren im allgemeinen erforderlich, noch eine Behandlung mit Säuren und eine Neutralisation vorzunehmen, um gute Ergebnisse, d.h. ein möglichst reines Phenol, zu erhalten.

Gemäss der vorliegenden Erfindung werden dagegen Phenole nur mit Ionenaustauschern behandelt. Eine anschliessende Destillation, sowie eine zusätzliche Behandlung mit Säuren und eine Neutralisation sind nicht erforderlich. Aufgrund der Angaben in der DE-OS 1 668 952 konnte nicht erwartet werden, dass die alleinige Behandlung von Phenolen mit Ionenaustauschern verbesserte Phenole ergibt, die sich mit Vorteil bei der Hydroxylierung mit Percarbonsäuren verwenden lassen.

Beispiele

Beispiel 1

Phenol ($C_6H_5OH$) wurde, wie weiter unten detailliert beschrieben, mit einem Unterschuss an Perpropionsäure umgesetzt und das anfallende Reaktionsgemisch destillativ aufgearbeitet. Nach Abtrennung von leichter und schwerer flüchtigen Komponenten wurde das nicht umgesetzte Phenol als flüssiges Produkt aus einer Rektifikationskolonne abgezogen und einer Behandlung mit einem Ionenaustauscher zugeführt.

Dazu wurde das abgetrennte Phenol mit einer Durchsatzgeschwindigkeit von 9 kg pro Stunde von unten nach oben durch eine stehende Edelstahlsäule (Durchmesser 100 mm, Höhe 500 mm) gepumpt, die durch einen Heizmantel auf 60°C Innentemperatur gehalten wurde. Die Säule war mit 1,74 kg eines makroporösen. schwach sauren Ionenaustauschers von Acrylsäure-Typ gefüllt, der zuvor mit Phenol gewaschen worden war (berechnet als Trockensubstanz).

Das aus der Säule austretende Produkt wurde in einem Sammelgefäss aufgefangen und unter Stickstoffüberlagerung bei 60 bis 70°C als Schmelze gehalten.

Zu 160 kg dieses Phenols wurden 20 kg frisches Phenol (Handelsware) zugesetzt und das so erhaltene Phenolgemisch nach gutem Durchmischen als Einsatzphenol für die nachstehend beschriebene Umsetzung verwendet.

Das vorbereitete Phenol wurde mit einer Durchsatzgeschwindigkeit von 11 kg pro Stunde dem ersten Reaktionsgefäss einer 4stufigen gerührten Kesselkaskade zugeführt, in das gleichzeitig 2,3 kg pro Stunde einer Lösung von Perpropionsäure in Benzol/Propionsäure eingebracht wurden. Die Perpropionsäure-Lösung enthielt 19,5 Gew.% Perpropionsäure, 13,3 Gew.% Propionsäure und 67 Gew.% Benzol. Die Reaktionsgefässe hatten ein nutzbares Volumen von je 1,7 l und wurden bei 42°C Innentemperatur gehalten. Das Reaktionsgemisch wurde durch Stickstoffüberlagerung auf das erste Reaktionsgefäss sowie durch den Produktstrom einmal durch alle 4 Reaktionsgefässe gedrückt.

Hinter dem 4. Reaktionsgefäss wurde im Reaktionsgemisch ein Gehalt von 0,04 Gew.% Perpropionsäure bestimmt, entsprechend einem Umsatz von 99% der eingesetzten Perpropionsäure.

Der Gehalt an Brenzcatechin im Reaktionsgemisch betrug 2,3 Gew.%, der Gehalt an Hydrochinon 1,4 Gew.%, entsprechend Selektivitäten von 55% für Brenzcatechin und 34% für Hydrochinon, jeweils bezogen auf umgesetzte Perpropionsäure.

Das Reaktionsgemisch wurde einer destillativen Aufarbeitung zugeführt, in deren Verlauf Benzol, Propionsäure, das überschüssige Phenol und die Reaktionsprodukte Brenzcatechin und Hydrochinon als reine Stoffe erhalten wurden. Das so abgetrennte Phenol wurde erneut, wie oben beschrieben, der Behandlung mit Ionenaustauscher zugeführt und wieder in die Reaktion eingesetzt.

Beispiel 2

(Vergleichsbeispiel zu Beispiel 1)

Es wurde gearbeitet wie in Beispiel 1, jedoch wurde das nach der Reaktion mit Perpropionsäure abgetrennte, nicht umgesetzte Phenol ohne die Behandlung mit Ionenaustauscher in dem im Beispiel 1 angegebenen Verhältnis mit Frischphenol versetzt und das so erhaltene Phenolgemisch unter den gleichen Bedingungen wie in Beispiel 1 mit Perpropionsäure umgesetzt.

In diesem Falle betrug der Umsatz 99,3%, bezogen auf die eingesetzte Perpropionsäure, und die Selektivität für die Bildung von Brenzcatechin 15% und für die Bildung von Hydrochinon 7%, jeweils bezogen auf umgesetzte Perpropionsäure.

Beispiel 3

200 g 4-tert. Butylphenol (Handelsware) werden bei 40°C in 500 ml Benzol gelöst. Unter Rühren und Stickstoff-Überlagerung wurden 130 g eines benzolfeuchten Ionenaustauschers (entsprechend 60 g trockenem Austauscher) auf der Basis von sulfoniertem und mit Divinylbenzol vernetztem Polystyrol in der $H^+$-Form zugesetzt und bei 40°C 30 Minuten gerührt.

Der Ionenaustauscher wurde anschliessend über eine Glasfritte abgetrennt; 500 g der als Filtrat erhaltenen Lösung, die 30 Gew.% 4-tert.-Butylphenol enthielt, wurden durch langsames Zutropfen unter Rühren und Stickstoffüberlagerung mit einer Lösung von 3,8 g Peressigsäure in 50 g Essigsäureethylester versetzt, wobei die Zutropfgeschwindigkeit so eingestellt wurde, dass die Temperatur nicht über 48°C anstieg.

Nach 120 Minuten wurde ein Persäure-Umsatz von 97,8% der eingesetzten Peressigsäure durch jodometrische Titration bestimmt. Nach gaschromatographischer Analyse enthielt das Reaktionsgemisch 6,1 g 4-tert. Butylbrenzcatechin, entsprechend einer Selektivität von 75%, bezogen auf die umgesetzte Peressigsäure.

Beispiel 4

(Vergleichsbeispiel zu Beispiel 3)

30 g 4-tert. Butylphenol wurden in 80 g Benzol gelöst und ohne Ionenaustauscher-Behandlung, aber sonst unter den Bedingungen des Beispiels 3, mit 08, g Peressigsäure in 9 g Essigsäureethylester bei 40°C umgesetzt.

Nach 120 Minuten wurde ein Umsatz von 98,2% der eingesetzten Peressigsäure bestimmt; der Gehalt an 4-tert. Butylbrenzcatechin betrug 1,03 g, entsprechend einer Selektivität von 59% der umgesetzten Peressigsäure.

Beispiel 5

200 g Phenol, welches als überschüssiges Phenol aus vorhergegangenen Umsätzen von Phenol mit Perpropionsäure durch Destillation bzw. Rektifikation zurückgewonnen worden war, wurde unter Stickstoff aufgeschmolzen und bei 70°C mit 30 g eines sauren Ionenaustauschers (Feuchtigkeitsgehalt 58%) auf der Basis von sulfonierten Styrol/Divinylbenzol-Copolymerisat (Natrium-Form) versetzt und bei 70°C 2 Stunden gerührt.

Anschliessend wurde der Ionenaustauscher über eine Glasfritte abfiltriert; von dem als Filtrat erhaltenen Phenol wurde 150 g unter Rühren und Stickstoff-Überlagerung bei 50°C durch langsames Zutropfen mit einer Lösung von 5 g Perpropionsäure in 17,3 g eines Gemisches aus 83,3 Gew.% Benzol und 16,7 Gew.% Propionsäure versetzt und bei 50–60°C umgesetzt.

Nach 60 Minuten wurde ein Umsatz von 97,4% der Perpropionsäure ermittelt, das Reaktionsgemisch enthielt 1,9 Gew.% Brenzcatechin und 1,2 Gew.% Hydrochinon, entsprechend Selektivitäten von 55% für die Bildung von Brenzcatechin und 34% für die Bildung von Hydrochinon, jeweils bezogen auf umgesetzte Perpropionsäure.

Beispiel 6

(Vergleichsbeispiel zu Beispiel 5)

Eine andere Probe des Einsatzphenoles für Beispiel 5 wurde ohne Ionenaustauscher-Behandlung unter sonst gleichen Bedingungen umgesetzt. Nach 60 Minuten Reaktionszeit wurde ein Umsatz von 98% der eingesetzten Perpropionsäure bestimmt, der Gehalt an Brenzcatechin und Hydrochinon entsprach Selektivitäten von 9% für Brenzcatechin und 2% für Hydrochinon.

Beispiel 7

250 g p-Ethylphenol (Handelsware) wurden bei 50°C unter Stickstoff aufgeschmolzen und unter Rühren mit 20 g eines feuchten schwach sauren Ionenaustauschers auf Acrylharzbasis ($H^+$-Form), (Feuchtigkeitsgehalt: 42%) versetzt.

Nach 60minütigem Rühren bei 50°C wurde der Ionenaustauscher über eine beheizte Glasfilternutsche abgetrennt; 150 g des als Filtrat angefallenen p-Ethylphenols wurden in einem 500-ml-Glasreaktionsgefäss mit Rührer, Rückflusskühler, Thermostatisierung, Temperaturmessung, Stickstoff-Überlagerung und Zutropfeinrichtung mit einer Lösung von 5 g Perisobuttersäure in 45 g 1,2-Dichlorpropan unter Rühren und Stickstoff-Überlagerung so langsam versetzt, dass die Temperatur nicht über 64°C anstieg. Nach einer Stunde Reaktionsdauer bei 60°C wurde ein Umsatz an Perisobuttersäure von 98,7% jodometrisch ermittelt; das Reaktionsgemisch enthielt 4,19 g Dihydroxyethylbenzole, entsprechend einer Hydroxylierungsselektivität von 64%.

Beispiel 8

(Vergleichsbeispiel zu Beispiel 7)

Das im Beispiel 7 mit dem Ionenaustauscher behandelte 4-Ethyl-phenol wurde direkt, d.h., ohne Ionenaustauscherbehandlung, unter sonst gleichen Bedingungen mit Perisobuttersäure umgesetzt. 260 g 4-Ethylphenol und 5,2 g Perisobuttersäure ergaben nach einer Stunde einen Umsatz von über 99% der Perisobuttersäure. Im Reaktionsgemisch wurden 3,5 g Dihydroxyethylbenzole gaschromatographisch analysiert, entsprechend einer Hydroxylierungsselektivität von 51%, bezogen auf die eingesetzte Perisobuttersäure.

**Patentansprüche**

1. Verfahren zur Herstellung mehrwertiger Phenole durch Hydroxylierung von Phenolen mit Percarbonsäuren, dadurch gekennzeichnet, dass man die zu hydroxylierenden Phenole in geschmolzener oder gelöster Form vor der Hydroxylierung ganz oder teilweise mit Ionenaustauschern behandelt, die saure und gegebenenfalls zusätzlich basische Gruppen enthalten.

2. Verfahren nach Anspruch 1, dadurch gekennzeichnet, dass man die Behandlung mit Ionenaustauschern vornimmt, die $HSO_3$- und/oder HOOC-Gruppen, sowie gegebenenfalls $NH_2$-, NH-Alkyl-, N-Dialkyl-, N-Alkylhydroxyalkyl- und/oder N-Diaryl-Gruppen enthalten, wobei diese Gruppen in freier Form oder ganz oder teilweise in Salzform vorliegen können.

3. Verfahren nach Ansprüchen 1 bis 2, dadurch gekennzeichnet, dass man die Behandlung mit festen Ionenaustauschern auf der Basis Acrylsäure-Divinylbenzol vornimmt, wobei die HOOC-Gruppen in freier Form oder ganz oder teilweise in Form ihrer Erdalkali- und/oder Alkalisalze vorliegen.

4. Verfahren nach Ansprüchen 1 bis 3, dadurch gekennzeichnet, dass das zu hydroxylierende Phenol eine aromatische Hydroxyverbindung ist, die sich von Benzol, Naphthalin, Phenanthren oder Anthracen ableitet und die mindestens ein freies Wasserstoffatom an einem aromatischen Kern aufweist.

5. Verfahren nach Anspruch 4, dadurch gekennzeichnet, dass das zu hydroxylierende Phenol ausser einer oder mehreren Hydroxylgruppen keine weiteren Substituenten aufweist.

6. Verfahren nach Anspruch 4, dadurch gekennzeichnet, dass das zu hydroxylierende Phenol ausser einer oder mehreren Hydroxylgruppen an dem oder den aromatischen Kernen als Substituenten einen oder mehrere, gleiche oder verschiedene aliphatische, cycloaliphatische, Phenyl- und/oder Naphthylreste; eines oder mehrere Fluor-, Chlor- und/oder Bromatome; eine oder mehrere $C_1$- bis $C_5$-Alkoxy-, $C_1$- bis $C_5$-Dialkylamino-, Carboxy-, Nitro-, Cyan-, Sulfonsäure- und/oder Carboalkoxygruppen, deren Alkoxyreste 1 bis 10 C-Atome aufweisen, enthalten.

7. Verfahren nach Ansprüchen 1 bis 6, dadurch

gekennzeichnet, dass man das zu hydroxylierende Phenol in geschmolzener oder gelöster Form mit Ionenaustauschern behandelt und die Behandlung bei Temperaturen im Bereich von 0 bis 200°C durchführt.

8. Verfahren nach Ansprüchen 1 bis 7, dadurch gekennzeichnet, dass die Behandlungszeit 1 bis 300 Minuten beträgt.

9. Verfahren nach Ansprüchen 1 bis 8, dadurch gekennzeichnet, dass man bei diskontinuierlicher Arbeitsweise 1 Gew.-Teil Ionenaustauscher zur Behandlung von 1 bis 1000 Gew.-Teilen zu hydroxylierendes Phenol anwendet oder bei kontinuierlicher Arbeitsweise pro Stunde 1 Gew.-Teil Ionenaustauscher mit 0,1 bis 1000 Gew.-Teilen zu hydroxylierendes Phenol in Kontakt bringt.

## Claims

1. Process for the preparation of polyhydric phenols by hydroxylation of phenols with percarboxylic acids, characterised in that, before the hydroxylation, all or some of the phenols to be hydroxylated are treated in a molten or dissolved form with ion exchangers which contain acid and if appropriate also basic groups.

2. Process according to Claim 1, characterised in that the treatment is carried out with ion exchangers which contain $HSO_3$ and/or HOOC groups and, if appropriate, $NH_2$, NH-alkyl, N-dialkyl, N-alkyl-hydroxyalkyl and/or N-diaryl groups, it being possible for these groups to be in the free form or completely or partly in salt form.

3. Process according to Claims 1 to 2, characterised in that the treatment is carried out with solid ion exchangers based on acrylic acid/divinylbenzene, the HOOC groups being in the free form or completely or partly in the form of their alkaline earth metal salts and/or alkali metal salts.

4. Process according to Claims 1 to 3, characterised in that the phenol to be hydroxylated is an aromatic hydroxy compound which is derived from benzene, naphthalene, phenanthrene or anthracene and has at least one free hydrogen atom on one aromatic nucleus.

5. Process according to Claim 4, characterised in that, besides one or more hydroxyl groups, the phenol to be hydroxylated contains no other substituents.

6. Process according to Claim 4, characterised in that, in addition to one or more hydroxyl groups on the aromatic nucleus or nuclei, the phenol to be hydroxylated contains, as substituents, one or more identical or different aliphatic, cycloaliphatic, phenyl and/or naphthyl radicals; one or more fluorine, chlorine and/or bromine atoms; one or more $C_1$- to $C_5$-alkoxy, $C_1$- to $C_5$-dialkylamino, carboxyl, nitro, cyano, sulphonic acid and/or carbalkoxy groups, the alkoxy radicals of which contain 1 to 10 C atoms.

7. Process according to Claims 1 to 6, characterised in that the phenol to be hydroxylated is treated in a molten or dissolved form with ion exchangers and the treatment is carried out at temperatures in the range from 0 to 200°C.

8. Process according to Claims 1 to 7, characterised in that the treatment time is 1 to 300 minutes.

9. Process according to Claims 1 to 8, characterised in that, in the case of a discontinuous procedure, 1 part by weight of ion exchanger is used for the treatment of 1 to 1,000 parts by weight of phenol to be hydroxylated, or, in the case of a continuous procedure, 1 part by weight of ion exchanger is brought into contact with 0.1 to 1,000 parts by weight of phenol to be hydroxylated, per hour.

## Revendications

1. Procédé de préparation de phénols polyvalents par hydroxylation de phénols avec des acides percarboxyliques, caractérisé en ce que, avant l'hydroxylation, on traite entièrement ou partiellement les phénols à hydroxyler sous forme fondue ou dissoute avec des échangeurs d'ions contenant des groupes acides et éventuellement, en outre, des groupes basiques.

2. Procédé suivant la revendication 1, caractérisé en ce qu'on effectue le traitement avec des échangeurs d'ions contenant des groupes $HSO_3$ et/ou HOOC, ainsi qu'éventuellement des groupes $NH_2$, NH-alkyle, N-dialkyle, N-alkylhydroxyalkyle et/ou N-diaryle, ces groupes pouvant se présenter sous forme libre ou encore entièrement ou partiellement sous forme de sels.

3. Procédé suivant les revendications 1 et 2, caractérisé en ce qu'on effectue le traitement avec des échangeurs d'ions solides à base d'acide acrylique/divinylbenzène, les groupes HOOC pouvant se présenter sous forme libre ou encore entièrement ou partiellement sous forme de leurs sels alcalino-terreux et/ou alcalins.

4. Procédé suivant les revendications 1 à 3, caractérisé en ce que le phénol à hydroxyler est un composé hydroxy aromatique dérivant du benzène, du naphtalène, du phénanthrène ou de l'anthracène et comportant au moins un atome d'hydrogène libre sur un noyau aromatique.

5. Procédé suivant la revendication 4, caractérisé en ce que, hormis un ou plusieurs groupes hydroxy, le phénol à hydroxyler ne comporte aucun autre substituant.

6. Procédé suivant la revendication 4, caractérisé en ce que, outre un ou plusieurs groupes hydroxy sur le ou les noyaux aromatiques, le phénol à hydroxyler, contient, comme substituants, un ou plusieurs radicaux phényle et/ou naphtyle, aliphatiques ou cycloaliphatiques, identiques ou différents; un ou plusieurs atomes de fluor, de chlore et/ou de brome; un ou plusieurs groupes alcoxy en $C_1$–$C_5$, dialkyl (en $C_1$–$C_5$) amino, carboxy, nitro, cyano, acide sulfonique et/ou carbalcoxy dont les radicaux alcoxy contiennent 1 à 10 atomes de carbone.

7. Procédé suivant les revendications 1 à 6, caractérisé en ce qu'on traite le phénol à hydroxyler sous forme fondue ou dissoute avec des échangeurs d'ions en ce qu'on effectue le traitement à

des températures se situant dans l'intervalle allant de 0 à 200°C.

8. Procédé suivant les revendications 1 à 7, caractérisé en ce que le temps de traitement est de 1 à 300 minutes.

9. Procédé suivant les revendications 1 à 8, caractérisé en ce que, lorsqu'on travaille en discontinu, on utilise 1 partie en poids d'échangeur d'ions pour le traitement de 1 à 1000 parties en poids du phénol à hydroxyler ou, lorsqu'on travaille en continu, on met, par heure, 1 partie en poids de l'échangeur d'ions en contact avec 0,1 à 1000 parties en poids du phénol à hydroxyler.